# EUROPEAN PATENT SPECIFICATION

(11) **EP 0 524 662 B1**
(45) Date of publication and mention of the grant of the patent: **12.11.1997**
(21) Application number: 92116352.3
(22) Date of filing: 27.06.1988
(51) Int. Cl.: A61M 1/36, F28F 9/16

(54) **Method for manufacturing a medical heat exchanger**
Verfahren zur Herstellung eines medizinischen Wärmetauschers
Procédé de fabrication d'un échangeur de chaleur médical

(30) Priority: 28.06.1987 JP 160094/87; 27.07.1987 JP 187040/87; 31.07.1987 JP 116765/87 U; 31.07.1987 JP 116766/87 U; 16.09.1987 JP 140073/87 U
(43) Date of publication of application: 27.01.1993
(62) Divisional of application: 88401641.1
(73) Proprietor: TERUMO KABUSHIKI KAISHA, Tokyo (JP)
(72) Inventor: Oshiyama, Hiroki, Terumo Kabushiki Kaisha, Shizuoka-ken (JP); Nogawa, Atsuhiko, Terumo Kabushiki Kaisha, Shizuoka-ken (JP)
(74) Representative: Casalonga, Axel

(56) References cited:
- EP-A- 0 081 118
- EP-A- 0 167 162
- DE-A- 1 479 420
- FR-A- 2 356 494
- US-A- 4 177 816
- US-A- 4 323 115

## Description

This invention relates to a method for the production of a heat exchanger for medical treatment, more particularly, a heat exchanger for medical treatment to be used in the extracorporeal circulation of blood for the purpose of maintaining the temperature of the blood at a desired level during the course of the circulation.

The extracorporeal blood circulation is generally employed as an auxiliary measure for surgery of the heart, particularly for cardiotomy. In the extracorporeal blood circulation as an auxiliary measure for cardiotomy, the blood drawn out of the patient's body is forwarded to an oxygenator, there to be oxygenated, and then returned in an oxygen-saturated state back to the patient's body. In the surgical operation performed on a complicated infantile cardiac deformation or on an adult aortic aneurysm, for example, the extremely low body temperature extracorporeal circulation method or the medial body temperature extracorporeal circulation method is employed. This blood circulation is effected by cooling the blood drawn out of the patient's body. In the extracorporeal body circulation of the nature under discussion, the blood drawn out of the patient's body must be kept at a prescribed temperature or cooled or heated. For this purpose, it has been customary to use a heat exchanger as inserted in the circuit for the extracorporeal blood circulation.

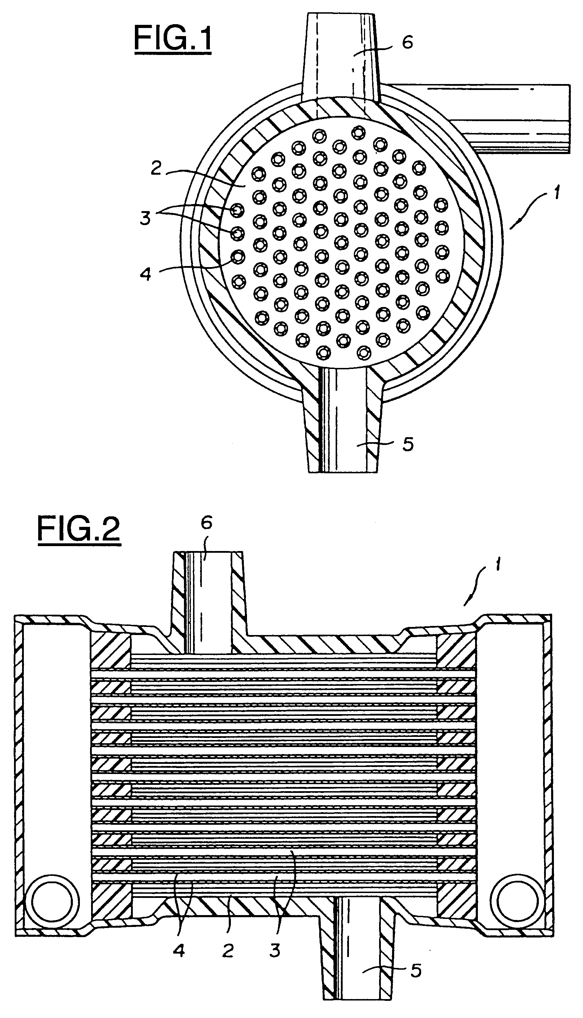

The heat exchangers developed to date for use in the extracorporeal blood circulation are widely varied in type. For example, a shell-and-tube exchanger 1 which, as illustrated in Figs. 1 and 2, comprises a first fluid passing space 2 and a multiplicity of heat-exchanging tubes 4 disposed inside the first fluid passing space 2 in the longitudinal direction of the first fluid passing space 2 and provided with an inner space 3 destined to form a second fluid passing space watertightly separated from the first fluid passing space 2 is a highly hopeful heat exchanger for medical treatment which effects very efficient exchange of heat and enjoys exceptional compactness of design. When the first fluid passing space 2 is formed in a cylindrical shape, a first fluid inlet tube 5 for introducing a first fluid into the first fluid passing space 2 and a first fluid outlet tube 6 for discharging the first fluid from the first fluid passing space 2 are adapted, as illustrated in Figs. 1 and 2, so as to be extended inwardly from outside substantially along the straight line passing the central part of a cross section perpendicular to the axis of the first fluid passing space 2 and consequently allowed to communicated with the first fluid passing space 2.

When the shell-and-tube exchanger 1 constructed as described above is used in effecting exchange of heat between the blood and the heat-exchanging medium by passing the heat-exchanging medium through the first fluid passing space 2 and the blood through the second fluid passing space, i.e., the inner spaces 3 of the heat-exchanging tubes 4, the exchange of heat can be accomplished substantially uniformly con the whole blood because the blood is distributed comparatively uniformly and allowed to keep a relatively constant contact with respect to the heat-exchanging medium. When the pressure loss during the introduction of the blood is large and the extracorporeal blood circulation lasts for a long time, there is a fair possibility that the blood will be coagulated inside the inner spaces 3 of the heat-exchanging tubes 4 and will consequently clog or constrict the heat-exchanging tubes 3. Conversely, when the exchange of heat is carried out by passing the blood through the first fluid passing space 2 and the heat-exchanging medium through the second fluid passing space or the inner spaces 3 of the heat-exchanging tubes 4, the aforementioned possibility of the blood conduits being clogged or constricted is substantially precluded and the pressure loss due to the introduction of the blood is repressed to a comparatively large extent. Since the first fluid inlet tube 5 and the first fluid outlet tube 6 are adapted, as described above, so as to be extended inwardly from outside substantially along the straight line passing the central part of a cross section perpendicular to the axis of the first fluid passing space 2 and consequently allowed to communicate with the first fluid passing space 2, the blood mainly advances toward the central part of the first fluid passing space 2 and consequently the flow of the blood inside the first fluid passing space 2 is not uniformized but is varied locally. In the region of relatively high blood flow, exchange of heat is effected to an unduly small extent because the blood does not sufficiently contact the heat-exchanging tubes 4 now passing the heat-exchanging medium inside the inner spaces 3 thereof. By contrast, in the region of relatively low blood flow, the exchange of heat is effected to an unduly large extent because the blood contacts the heat-exchanging tubes 4 more than is normally required. Where the heat-exchanging tubes 4 are distributed throughout the whole blood passing space 2 as illustrated in Figs. 1 and 2, the unfavorable situation mentioned above grows in conspicuity because the blood introduced through the blood inlet tube 5, on entering the blood passing space 2, comes into direct contact with the heat-exchanging tubes 4 and, consequently, the blood flow is not given a chance enough to be uniformly distributed throughout the entire blood passing space 2. The shell-and-tube exchanger which is incapable of effecting exchange of heat uniformly on the whole blood being passed therethrough hardly deserves high esteem because it has the drawback of impairing the uniformity of blood temperature distribution, suffering the exchange of heat on the blood to proceed excessively or insufficiently, and bringing about adverse effects upon the blood components.

Further, the conventional heat exchanger is so constructed that a heat-exchanging medium inlet port for introducing the heat-exchanging medium into the heat exchanger and a heat-exchanging medium outlet port for discharging the heat-exchanging medium from within the heat exchanger are integrally formed with a housing of the heat exchanger and are fixed on the housing. A connection tube which leads out of a heat-exchanging medium temperature controller communicating with the heat-exchanging medium inlet port and the heat-exchanging medium outlet port is generally large in diameter and hard to the touch. The connection between the coupler disposed on the heat-exchanging medium inlet port or the heat-exchanging medium outlet port and the coupler disposed at the leading end of the connection tube of the heat-exchanging medium temperature controller is obtained only with difficulty. Moreover, there is a risk that this connection will be disrupted in consequence of a deviation of the positional relationship between the heat exchanger and the heat-exchanging medium temperature controller during the course of operation.

EP-A-81118 (TERUMO) discloses a method for the production of a heat exchanger for the blood under extra corporeal treatment comprising the steps of :
- introducting a multiplicity of slender heat-exchanging tubes into a cylindrical housing possessing a heat-exchanging medium inlet port, a heat-exchanging medium outlet port, a blood inlet port and a blood outlet port;
- fitting first and second sealing members to the end parts of the housing;
- injecting a potting compound into the cylindrical housing with the end parts of the slender heat-exchanging tubes kept in closed state thereby forming partition walls for fixing each end part of the slender heat-exchanging tubes to the cylindrical housing;
- and removing the sealing members.

In this method as disclosed, the correct arrangement of the heat exchanging tubes is maintained by the mere thickness of supplemental plastic tubes which are mounted at the ends of the heat-exchanging tubes, thus leading to a certain degree of imprecision.

Furthermore, the disclosed method provides for the use of specific means closing the end of the tubes in a preliminary step of the method. Upon completion of the manufacture it is then further necessary to cut the ends of the heat-exchanging tubes which is difficult to execute with high degree of precision.

An object of this invention, therefore, is to provide an improved method for the production of a heat exchanger for medical treatment, said method being particularly easy to execute and giving a very precise result.

As claimed the method of the present invention for manufacturing a medical heat exchanger, comprises the steps of introducing a multiplicity of slender heat-exchanging tubes into a cylindrical housing possessing a heat-exchanging medium inlet port, a heat-exchanging medium outlet port, a blood inlet port and a blood outlet port; fitting a first and second sealing members to the end parts of the housing; injecting a potting compound into the cylindrical housing with the end parts of the slender heat-exchanging tubes kept in closed state thereby forming partition walls for fixing each end part of the slender heat-exchanging tubes to the cylindrical housing; and removing the first and second sealing members.

According to the invention, the potting compound is injected through the blood inlet port and the blood outlet port which are disposed at positions between said medium ports.

Furthermore, the sealing members comprise elastic sealing members capable of closing the end parts of the slender heat-exchanging tubes and are attached at respective positions between said medium port and blood port to both end parts of the cylindrical housing.

The slender heat-exchanging tubes are introduced into the housing aided with a slender tube distributing device possessing a multiplicity of holes for insertion of the slender heat-exchanging tubes.

End plates are also finally fitted on the opposite ends of the cylindrical housing.

The heat exchanger for medical treatment is thus produced by disposing a multiplicity of heat-exchanging tubes mutually separated inside a cylindrical housing possessing closed opposite ends in the longitudinal direction or the housing, partition walls disposed at the opposite end parts of the heat-exchanging tubes to hold the heat-exchanging tubes fast watertightly on the lateral wall of the housing without closing the openings of the heat-exchanging tubes and, at the same time, partition the interior or the housing into three spaces, a blood inlet tube and a blood outlet tube communicating with a blood passing space formed in the central part of the housing by the two partition walls, the lateral wall of the housing, and the outer walls of the heat-exchanging tubes, and a heat-exchanging medium inlet tube communicating with one of two heat-exchanging medium passing spaces formed at the end parts of the housing communicating with the inner spaces of the heat-exchanging tubes watertightly separated from the blood passing space and a heat-exchanging medium outlet tube to communicate with the other heat-exchanging medium passing space. The heat exchanger may be such that the blood inlet and outlet tubes severally extend inwardly from outside substantially along straight lines perpendicular to the longitudinal direction of the housing and tangent to the peripheral surface of the housing The heat exchanger for medical treatment may be such that the blood inlet tube communicates with the blood passing space in the proximity of one of the partition walls and the blood outlet tube communicates with the blood passing space in the proximity of the other partition wall. The heat exchanger for medical treatment may be such that the blood inlet tube and the blood outlet tube assume a positional relation such that they are mounted at about 180° from each other around the peripheral surface of the blood passing space. The heat exchanger for medical treatment may be such that the two empty space portions destitute of arrangement of heat-exchanging tubes within the blood passing space occupy a total volume of less than 40% of the blood passing space.

The heat exchanger for medical treatment may be such that the cylindrical housing comprises at lease one rib formed on the inner wall surface of the housing extending parallelly to the slender heat-exchaning tubes to retard the flow of blood between the slender heat-exchanging tubes and the inner wall surface of the housing.

The method of the present invention for the production of a heat exchanger for the blood under treatment by the extracorporeal circulation, may also comprise the steps of forming a cylindrical housing possessing heat-exchanging medium ports disposed each one at the opposite end parts thereof and a blood inlet port and a blood outlet port disposed at positions between the medium ports, attaching a first sealing member at a position between those of the medium ports and the blood ports which are located at one end part of the cylindrical housing, inserting through the other end part of the cylindrical housing into the cylindrical housing a slender tube distributing device possessing a multiplicity of holes for insertion of slender, heat-exchanging tubes and a multiplicity of slender heat-exchanging tubes, attaching a second sealing member at a position between those of the medium ports and the blood ports which are located at the other end part of the cylindrical housing, injecting a potting compound through the blood port on one end part side and the blood port on the other end part side of the cylindrical housing with the end parts of the slender heat-exchanging tubes kept in a closed state thereby forming partition walls for fixing the opposite end parts of the slender heat-exchanging tubes to the cylindrical housing, removing, the first sealing member and the second sealing member, and fitting end plates one each to the opposite ends of the cylindrical housing.

The insertion of the slender tube distributing device possessing a multiplicity of holes for insertion of slender heat-exchanging tubes and a multiplicity of slender heat-exchanging tubes through the other end part of the cylindrical housing into the cylindrical housing is carried out, for example, while keeping the cylindrical housing set upright on the one end part of the cylindrical housing to which the first sealing member has been attached. Otherwise, the insertion of the slender tube distributing device possessing a multiplicity of holes for insertion of slender heat-exchanging tubes and a multiplicity of slender heat-exchanging tubes through the other end part of the cylindrical housing into the cylindrical housing is carried out, for example, by a procedure which comprises first inserting through the other end part of the cylindrical housing into the cylindrical housing the slender tube-distributing device possessing a multiplicity of holes for insertion of slender heat-exchanging tubes and subsequently inserting the multiplicity of slender heat-exchanging tubes. The formation of partition walls for fixing the opposite end parts of the slender heat-exchanging tubes to the cylindrical housing by the injection of a potting compound through the blood port on one end part side and the blood port on the other end part side of the cylindrical housing with the end parts of the slender heat-exchanging tubes kept in a closed state is accomplished, for example, by injecting the potting compound through the blood port on one end part side of the cylindrical housing, then turning the cylindrical housing upside down, injecting the potting compound through the blood port on the other end part side of the cylindrical housing, and allowing the injected portions of the potting compound to set and give rise to partition walls. Further, the formation of partition walls for fixing the opposite end parts of the slender heat-exchanging tubes to the cylindrical housing by the injection of a potting compound through the blood port on one end part side and the blood port on the other end part side of the cylindrical housing with the end parts of the slender heat-exchanging tubes kept in a closed state is accomplished, for example, by injecting the potting compound through the blood port on one end part side of the cylindrical housing, allowing the injected portion of the potting compound to set, then removing the second sealing member and the slender tube-distributing device, attaching the second sealing member again in place turning the cylindrical housing upside down, injecting the potting compound through the blood port on the other end part side of the cylindrical housing with the end parts of the slender heat-exchanging tubes kept in a closed state, and allowing the injected portion of the potting compound to set. The step of removing the first sealing member and the second sealing member and the step of attaching end plates to the opposite ends of the cylindrical housing are carried out, for example, by a procedure which comprises first removing the first sealing member and the second sealing member and subsequently attaching each end plate to the opposite end parts of the cylindrical housing. Further, the step of removing the first sealing member and the second sealing member and the step of attaching end plates to the opposite ends of the cylindrical housing are carried out, for example, by a procedure which comprises a step of removing either the first sealing member or the second sealing member and attaching one of the end plates to the end part from which the sealing member has been removed and step of removing the remaining sealing member and attaching the remaining end plate to the end part from which the remaining sealing member has been removed. Further, the first sealing member comprises preferably an elastic sealing member kept in place by a retaining device.

### BRIEF DESCRIPTION OF THE DRAWINGS

Fig. 1 is a cross section taken through a conventional heat exchanger perpendicularly to the axis thereof to illustrate the construction thereof,
Fig. 2 is a cross section taken through the conventional heat exchanger in the directon of the axis thereof,
Fig. 3 is a partially cutaway perspective view illustrating one embodiment of a typical heat exchanger for medical treatment manufactured by the method of the present invention,
Fig. 4 is a cross section taken through Fig. 3 along the line IV-IV,
Fig. 5 is a cross section taken through Fig. 3 along the line V-V,
Fig. 6 is a partially sectioned front view of an oxygenator incorporating therein the heat exchanger manufactured by the method of the present invention,
Fig. 7 is a perspective view of the oxygenator illustrated in Fig. 6,
Fig. 8 is a partially cutway front view illustrating another embodiment of the heat exchanger manufactured by the method of the present invention,
Fig. 9 is a partially enlarged cross section of Fig. 8,
Fig. 10 is a drawing for explaining the manufacturing process of the heat exchanger in accordance with the present invention,
   and
Fig. 11 is a partially enlarged cross section of Fig. 10.

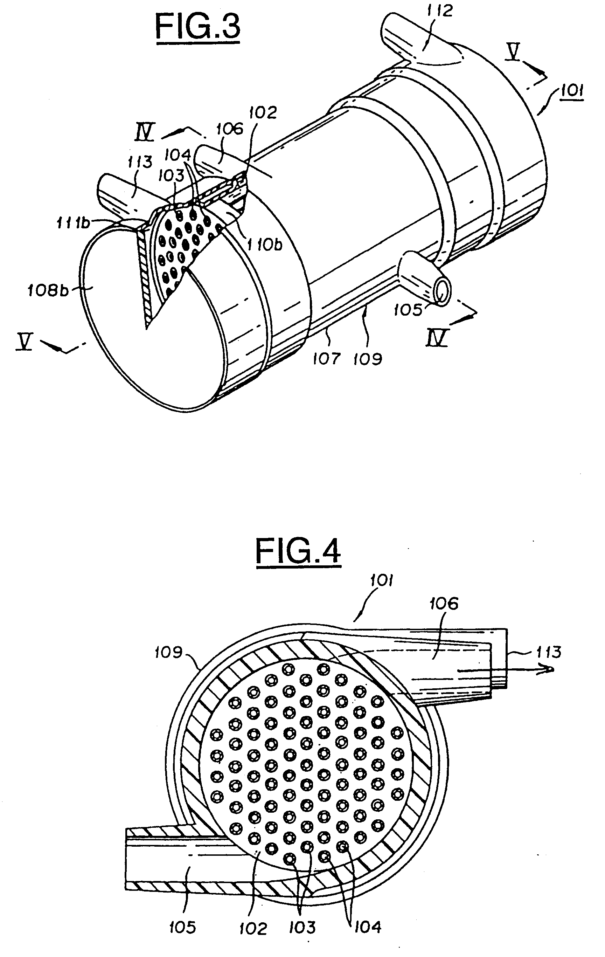

A heat exchanger 101 for medical treatment, as illustrated in Figs. 3 to 5, comprises a cylindrical blood passing space 102 and a multiplicity of heat-exchanging tubes 103 disposed inside the cylindrical blood passing space 102 along the longitudinal direction of the blood passing space 102 and each provided with an inner space watertightly separated from the blood passing space 102 and effects exchange of heat across the walls of the heat-exchanging tubes 103 between, the blood passed through the blood passing space 102 and a heat-exchanging medium passed through the inner spaces 104 of the heat-exchanging tubes 103. This heat exchanger 101 has as the salient characteristic thereof the fact that a blood inlet tube 105 for introducing blood into the blood passing space 102 and a blood outlet tube 106 for discharging the blood from the blood passing space 102 are severally extended inwardly from outside substantially along straight lines perpendicular to the longitudinal direction of the blood passing space 102 and tangent to the peripheral surface of the blood passing space 102 and allowed to communicate with the blood passing space 102.

When the blood inlet tube 105 and the blood outlet tube 106 are severally extended inwardly from outside substantially along straight lines perpendicular to the longitudinal direction of the blood passing space 102 and allowed to communicate with the blood passing space 102 as described above, the blood introduced through the blood inlet tube 105 is induced to assume a flow tending to revolve inside the blood passing space 102 along the periphery of the blood passing space 102 and, the blood passed through the interior of the blood, passing space 102 is caused to come into uniform contact with substantially all the heat-exchanging tubes 103 disposed inside the blood passing space 102 except mainly for a specific group of heat-exchanging tubes 103 which are located in the central part of the blood passing space 102, namely on a roughtly straight line connecting the communicating entrance to the blood inlet tube 105 and the communicating entrance to the blood outlet tube 106. Thus, the exchange of heat is attained uniformly within the blood passing space 102 without entailing any locally excessive or insufficient exchange of heat. Further in the heat exchanger of this invention, since the blood is passed outside the heat-exchanging tubes 103, it is neither compelled to find only a limited flow path during the course of introduction thereof into the blood passing space 102 nor forced to suffer from any large pressure loss or entail any heavy damage to the blood components.

The heat exchanger for medical treatment in the present embodiment is so constructed that inside a cylindrical housing 109 of closed opposite ends formed of a housing proper 107 and end plates 108a, 108b closing the open opposite ends thereof as illustrated in Figs. 3 to 5, the multiplicity of heat-exchanging tubes 103 are disposed mutually separated along the longitudinal direction of the housing 109 and partition walls 110a, 110b disposed one each at the opposite end parts of the plurality of heat-exchanging tubes 103 retain the heat-exchanging tubes 103 watertightly to the lateral wall of the housing 109 without closing the openings of the heat-exchanging tubes 103. At the same time, these partition walls 110a, 110b serve the purpose of partitioning the interior of the housing 109 into three empty spaces. The central portion of the housing 109 enclosed with the two partition walls 110a, 110b, the lateral wall of the housing 109, and the outer walls of the heat-exchanging tubes 103 constitutes itself the blood passing space 102 and the two end portions of the housing 109 watertightly separated from the blood passing space 102 and enclosed with the partition walls 110a, 110b and the end part walls and the lateral wall of the housing 109 constitute themselves the heat-exchanging medium passing spaces 111a, 111b. These two heat-exchanging medium passing spaces 111a, 111b both communicate with the inner spaces 104 of the heat-exchanging tubes 103 which are watertightly separated from the blood passing space 102. In the construction, formed as described above, the blood inlet tube 105 and the blood outlet tube 106 are allowed to communicated with the blood passing space 102 and the heat-exchanging medium outlet tube 112 to communicate with the heat-exchanging medium passing space 111a and the heat-exchanging medium inlet tube 113 with the other heat-exchanging medium passing space 111b.

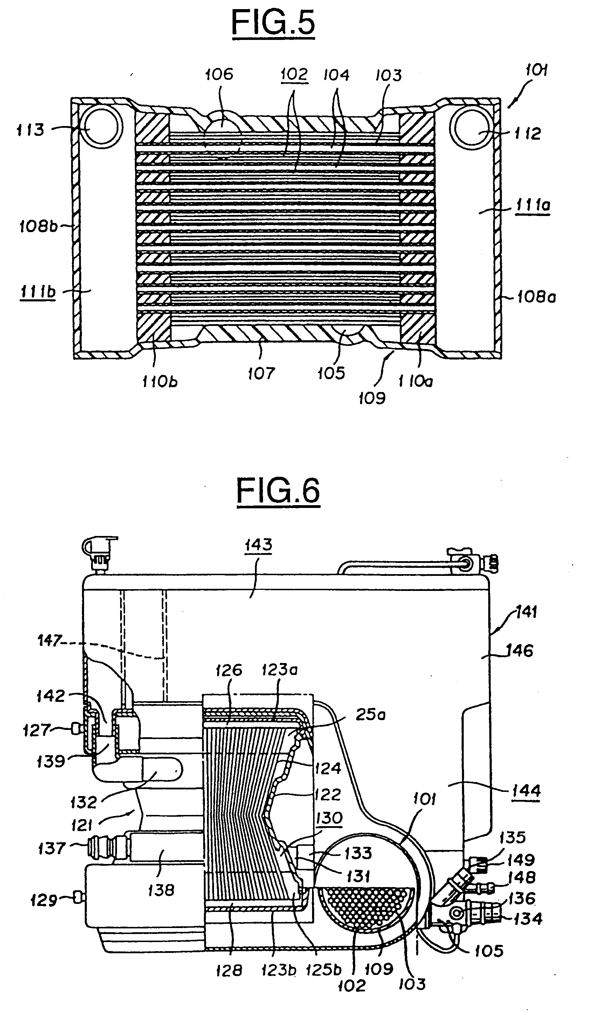

Further, in the heat exchanger 101 of the present embodiment for medical treatment, the blood inlet tube 105 and the blood outlet tube 106 are severally extended inwardly from outside substantially along straight lines perpendicular to the longitudinal direction of the housing 109 and tangent to the peripheral surface of the housing 109, namely substantially along straight lines perpendicular to the longitudinal direction of the blood passing space 102 and tangent to the peripheral surface of the blood passing space 102, and consequently allowed to communicate with the blood passing space 102. The blood inlet tube 105 and the blood outlet tube 106 which are extended inwardly from outside need not fall exactly on the straight lines perpendicular to the longitudinal direction of the blood passing space 102 and tangent to the peripheral surface of the blood passing space 102 but may deviate from the straight lines to an extent such that they will not be prevented from effectively imparting to the blood passed through the blood passing space 102 a flow along the peripheral surface of the blood passing space 102. Further, the blood inlet tube 105 and the blood outlet tube 106 are only required, at least in the portions thereof immediately before their points of communication with the blood passing space 102, to run roughly along the straight lines perpendicular to the longitudinal direction of the blood passing space 102 and tangent to the peripheral surface of the blood passing space 102. For the subsequent portions thereof, the direction is a matter for arbitrary decision. The positions at which the blood inlet tube 105 and the blood outlet tube 106 are to be located are not specifically defined. For the purpose of ensuring effective exchange of heat between the heat-exchanging medium passed through the inner spaces of the heat-exchanging tubes 103 inserted inside the blood passing space 102 and the blood passed through the blood passing space 102, they must be allowed to communicate with the blood passing space 102 at mutually separated positions. Desirably, as illustrated in Figs. 3 to Fig. 5, the blood inlet tube 105 is allowed to communicate with the blood passing space 102 near one of the partition walls 111a and 111b and the blood outlet tube 106 to communicate with the blood passing space 102 near the other partition walls 111b or 111a. Further, the blood inlet tube 105 and the blood outlet tube 106 are desired to assume a positional relation such that they are mounted at about 180° from each other around the peripheral surface of the blood passing space 102 as illustrated in Figs. 3 and 4.

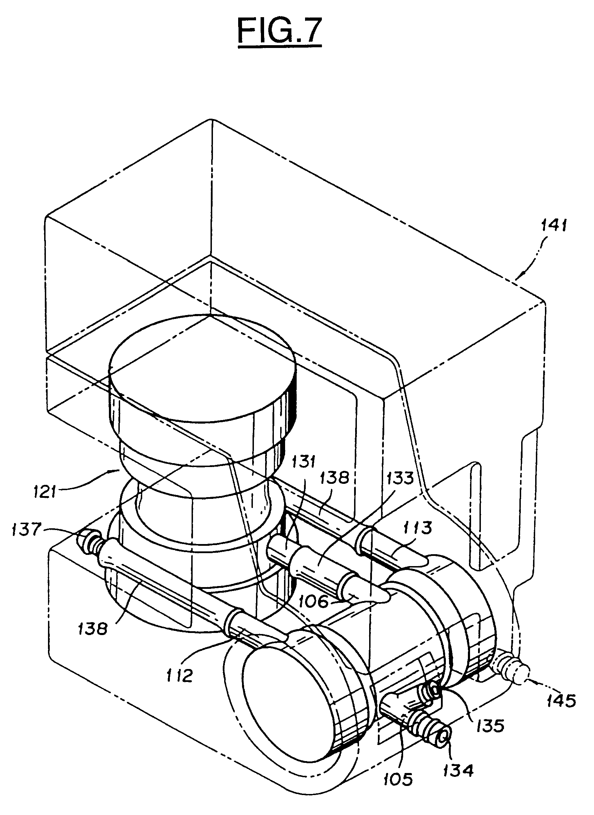

The heat exchanger 101 for medical treatment of the present embodiment which is constructed as described above is put to use as incorporated suitably in a varying extracorporeal circulation circuit. Since it is featured by exceptional compactness of design asnd high performance, it can be advantageously utilized as integrated with an oxygenator and a blood storing tank and operated as an oxygenator system as illustrated in Figs. 6 and 7, for example.

In the embodiment illustrated in Figs. 6 and 7, an oxygenator 121 is provided with a housing which comprises a cylindrical housing body 122 and fitting covers 123a, 123b closing the open opposite end parts of the housing body 122. Inside the housing, a multiplicity of hollow fiber membranes 124 are paralelly disposed mutually separated along the longitudinal direction of the housing and distributed cross-sectionally throughout the entire interior of the housing. The opposite end parts of these hollow fiber membranes 124 are watertightly retained on the housing body 122 by means of partition walls 125a, 125b, with the openings of the opposite end parts kept in an opened state. A gas inlet port 127 is disposed so as to communicate with a gas inlet space 126 defined by the fitting cover 123a, the housing body 122, and the partition wall 125a and allowed to communicate with the inner spaces of the hollow fiber membranes and a gas passing port 129 is disposed so as to communicate with a gas passing space 128 defined by the other fitting cover 123b, the housing body 122, and the partition wall 125b and allowed to communicate with the inner spaces of the hollow fiber membranes. Further, a blood inlet tube 131 and a blood outlet tube 132 are disposed so as to communicate with a blood chamber 130 formed of the inner wall of the housing body 122, the two partition walls 125a, 125b, and the outer walls of the hollow fiber membranes 124.

The oxygenator 121 illustrated in the present embodiment is of a type which effects exchange of gas by blowing an oxygen-containing gas such as air into the inner spaces of the hollow fiber membranes and passing blood outside the hollow fiber membranes 114. Otherwise, the oxygenator may be constructed in a type which effects the exchange of gas by passing blood in the inner spaces of the hollow fiber membranes and a passing the oxygen-containing gas outside the hollow fiber membranes. Alternatively, an oxygenator of a type using flat gas-exchange membranes is also usable. In all these types of oxygenator, particularly desirable is the type which passes the blood outside the hollow fiber membranes as illustratd in the present embodiment. Since the oxygenator of this type has any sparing pressure loss, the oxygenator system using this oxygenator does not need a blood pump in front of the oxygenator inserted in the path of circulation circuit. It has only to rely upon the removal of blood from a patient's body owing to the head of pressure to obtain required flow of the blood to the oxygenator and further to the blood storing tank.

The blood outlet tube 106 of the heat exchanger 101 for medical treatment is allowed to communicate watertightly with the blood inlet tube 131 of the oxygenator 121 through the medium of a connection tube 133 as illustrated in Fig. 7. The watertight connection of the connection tube 133 to the blood inlet tube 131 of the oxygenator 121 and to the blood outlet tube 6 of the heat exchanger 101 for medical treatment is attained by tight fitting by the use of a screw, a taper, or an O-ring or by tight adhesion by means of ultrasonic waves or high-frequency waves by with adhesive agent. Of course, it is permissible to connect the blood inlet tube 131 of the oxygenator 121 directly and watertightly to the blood outlet tube 6 of the heat exchanger 101 for medical treatment by similar means of connection. The exchanger for medical treatment in the present embodiment and illustrated in Figs. 3 to 5 are substantially identical, though they differ slightly with respect to the positions at which the blood inlet tube 105 and the blood outlet tube are located. The blood inlet tube 105 in the heat exchanger 101 for medical treatment in the present embodiment is provided with a cardiotomy inlet port 135 for introduction of the blood shed during the course of surgical operation in addition to a blood inlet port 134 for connection to the extracorporeal circulation path. The blood inlet tube 105 of this heat exchanger 101 is provided with a temperature measuring probe insertion hole 136. To the heat-exchanging medium inlet tube 113 and the heat-exchanging medium outlet tube 112, flexible extension tubes 138 provided at the leading end part thereof with a water inlet port (not shown) or a water outlet port 137 are connected.

In the meantime, a blood inlet 142 of a blood storing tank 141a is connected watertightly with a connection tube 139 to the blood outlet tube 132 of the oxygenator 121. The watertight connection of the connection tube 139 to the blood outlet tube of the oxygenator 121 and to the blood outlet tube 142 of the blood storing tnak 141 is attained in the same manner as in the watertight connection of the connection tube 133 to the blood inlet tube 131 of the oxygenator 121 and to the blood outlet tube 106 of the heat exchanger 101 for medical treatment.

With a housing 146 made of rigid material and provided with a blood inlet 142, a blood inlet part 143 communicating with the blood inlet 142 and possessing a bottom surface having substantially no head of pressure from the blood inlet 142, a blood storing part 144 communicating with the blood inlet part 143 and possessing a bottom surface destined to fall gradually from the blood inlet part 143, and a blood outlet 145 disposed below the blood storing part 144, the blood storing tank 141 which is conncected to the oxygenator 121 is formed by disposing a defoaming member 147 across the entire width of the blood flow path of the blood inlet part 143. This blood storing tank 141 is provided not only with the blood outlet 145 connected to the extracorporeal circulation path but also with a cardio-pregear part 148 destined to be connected to the path for forwarding blood to the cardiac vein and adapted to communicated with the lower part of the blood storing part 144. It is further provided a temperature measuring probe insertion port 149 intended to measure the temperature of blood within the blood storing tank 141.

In the oxygenator system which integrates the heat exchanger 101 for medical treatment and the blood storing tank 114 with the oxygenator 121 as described above, the blood withdrawn from a patient's body flows into the heat exchanger 101 via the blood inlet tube 105. Since the blood inlet tube 105 and the blood outlet tube 106 are severally extended inwardly from outside substantially along the straight lines perpendicular to the longitudinal direction of the blood passing space 102 and tangent to the peripheral surface of the blood passing space 2 and allowed to communicate with the blood passing space 102 as described above, the blood introduced through the blood inlet tube 105 into the blood passing space 102 is induced to generate a flow tending to revolve inside the blood passing space 102 along the inner peripheral surface of the blood passing space 102 and the blood passed through the interior of the blood passing space 102 is brought into uniform contact with substantially all the heat-exchanging tubes 103 disposed inside the blood passing space 102, except mainly for the specific group of heat-exchanging tubes 103 which are located in the central part of the blood passing space 102, namely on the roughly straight line connecting the point of communication with the blood inlet tube 105 and the point of communication with the blood outlet tube 106. As a result, the exchange of heat across the walls of the heat-exchanging tubes within the blood passing space 102 between the blood and the water, i.e. a heat-exchanging medium, which is introduced through the heat-exchanging medium inlet tube 113 into the heat-exchanging medium passing space 111a, passed through the inner spaces 104 of the heat-exchanging tubes 103 to the heat-exchanging medium passing space 111b, and discharged through the heat-exchanging medium outlet tube 112 is carried out efficiently and uniformly. The blood which has been heated or cooled to a desired temperature is led out of the heat exchanger 101 through the blood outlet tube 106 and then forwarded to the oxygenator 121 through the blood inlet tube 131 of the oxygenator 121 communicating watertightly with the blood outlet tube 106. The blood which has flowed into the oxygenator 121 via the blood inlet tube 131, while flowing through the blood chamber 130, exchanges gas through the medium of the walls of the hollow fiber membranes 124 with the oxygen-containing gas which is flowing through the inner spaces of the hollow fiber membranes 124. Consequently, the blood is divested of excess carbon dioxide gas and instead replenished with fresh oxygen. The blood which has been oxygenated flows out of the blood outlet tube 132 of the oxygenator 121 and then continues its flow into the blood storing tank 141 through the blood inlet 142 of the blood storing tank 141. The blood which has blowed from the blood inlet 142 to the blood inlet part 143 communicating therewith and reached the defoaming member 147 disposed in route to the blood inlet part 143 is defoamed during the course of its passage through the defoaming member 147 by the fact that the bubbles entrained by the blood come into contact with the foam cells of the defoaming member 147, gradually agglomerate, and depart from the blood and move to the upper empty space inside the blood storing tank 141. The defoamed blood further moves to the blood storing part 144, remains temporarily in the blood storing part 144, releases itself through the blood outlet 145 disposed below the blood storing part 144, and finds its way to the patient's body.

As slender heat-exchanging tubes, the heat exchanger of the present invention is desirably provided in the housing thereof with about 10 to 2,000, preferably about 50 to 1,000, metallic tubes of high thermal conductivity (such as, for example, stainless steel tubes, aluminum tubes, or copper tubes) having an inside diameter in the range of 0.5 to 10 mm, preferably 2 to 5 mm. These slender tubes are accommodated in the housing parallelly to the axial direction of the housing. The blood introduced through the blood inlet, therefore, flows inside the housing in such a direction as to traverse the slender tubes. The slender tubes are separated by a fixed distance. This distance, though variable with the outside diameter of slender tube or the inside diameter of housing, for example, generally falls approximately in the range of 0.2 to 4 mm, preferably 0.8 to 2 mm.

The housing is made of any of various materials such as polycarbonate, acryl-styrene copolymer, and acryl-butylene-styrene copolymer. The seal members used for sealing the opposite ends of the housing are discs possessed of an outer contour substantially equal to the inner contour of the end parts of the housing and made of any of various materials such as polycarbonate, acryl-styrene copolymer, and acryl-butylene-styrene copolymer. These seal members are fixed watertightly to the end part of the housing by means of adhesion with adhesive agent or solvent or fusion with high-frequency waves, ultrasonic waves, or induction heating.

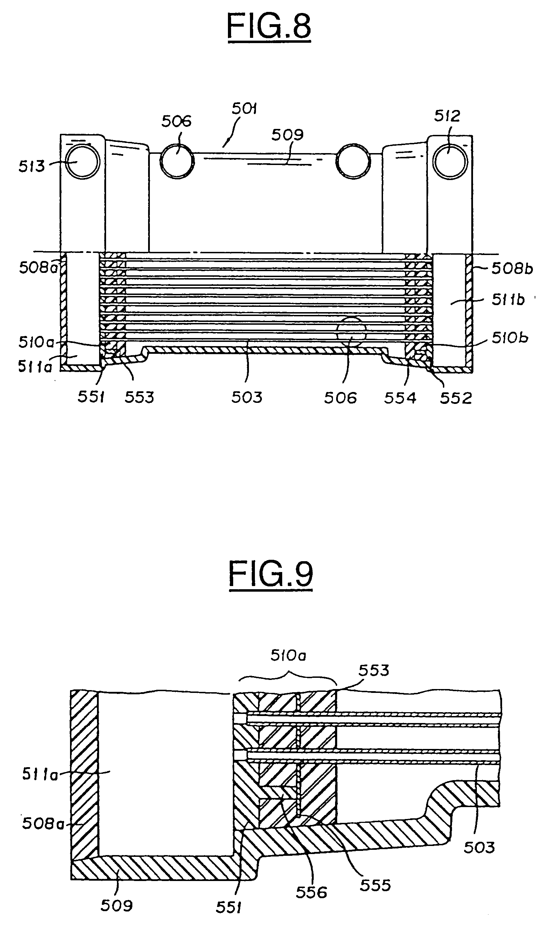

Fig. 8 and Fig. 9 illustrate a further embodiment manufactured by the method of the present invention. The difference between the heat exchanger of this embodiment and that of the embodiment illustrated in Fig. 3 to Fig. 5 consists mainly in the construction of partition walls 510a, 510b. In Fig. 8 and Fig. 9, the parts which have equivalents in Fig. 3 to Fig. 5 are denoted by like reference numerals plus 400. Fig. 9 is a magnified cross section illustrating a partition wall and adjacent parts of the heat exchanger of Fig. 8.

A cylindrical housing 509 of a heat exchanger 501 accommodate therein a multiplicity of slender heat-exchanging tubes 503. The housing 509 and the slender tubes 503 are similar to those already described. In the present embodiment, partition walls 510a, 510b are formed respectively of perforated plates 551, 552 and potting compounds 553, 554. The partition walls will be described specifically with reference to Fig. 9. The perforated plate 551 possesses a multiplicity of holes each having an inside diameter larger than the outside diameter of slender tube 503 at one end thereof and an inside diameter smaller than the outside diameter of slender tube 503 at the other end thereof. In the embodiment of Fig. 9, the perforated plate 551 possesses holes which have an inside diameter converged from one end to the other thereof so as to decrease past the outside diameter of the slender tubes 503 halfway along the wall thickness of the perforated plate. The end parts of the slender tubes 503 are inserted into these holes in the perforated plate. Further, the perforated plate 551 is provided with a plurality (at least two) ribs 556 serving the purpose of keeping a slender tube dispersing plate 555 apart from the perforated plate 551. The slender tube dispersing plate 555 is intended to impart a fixed pattern to the bundle of slender tubes 503 in a dispersed state and, therefore, is provided with a multiplicity of holes fit for insertion of the slender tubes 503. The provision of the slender tube dispersion plate and the ribs, though not an indispensable requirement, proves to be desirable in the sense that it ensures uniform dispersion of the slender tubes 503. The potting compound 553 fixes the slender tubes 503 watertightly to the perforated plated 551 and the perforated plate 551 similarly watertightly to the housing 509. The slender tube dispersing plate 555 is completely buried in the potting compound. The complete embedment in the potting compound brings about an advantageous effect in preventing the blood under treatment from coming into direct contact with the dispersing plate. The partition walls 510b and the adjacent parts are similarly constructed.

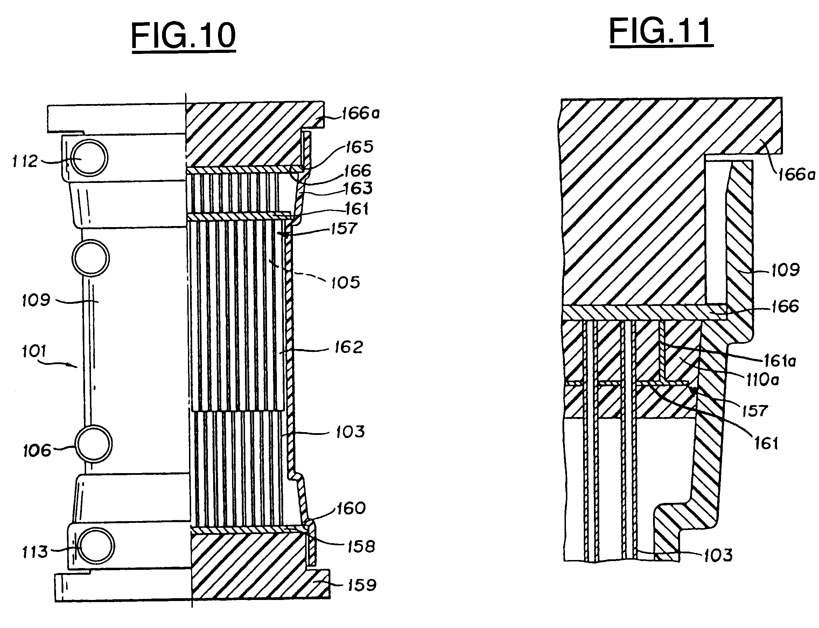

Now, the method employed for the production of the heat exchanger for medical tretment of the present invention illustrated in Fig. 3 to Fig. 5 will be described below with reference to Fig. 10 and 11.

The method for the production contemplated by the invention comprises the steps of forming a cylindrical, housing possessing heat-exchanging medium ports 112, 113 disposed one each at the opposite end parts thereof and a blood inlet port 105 and a blood outlet port 106 disposed at positions between the medium ports 112, 113, attaching a first sealing member 158 at a position between the medium port (medium inlet port 113, for example) and the blood port (blood outlet port 106, for example) located at one end part of the cylindrical housing 109, inserting through the other end part of the cylindrical housing 109 into the cylindrical housing a slender tube distributing or dispersing device 157 possessing a multiplicity of holes fit for insertion of slender heat-exchanging tubes and a multiplicity of slender heat-exchanging tubes 103, attaching a second sealing member 166 at a position between the medium port (medium outlet port 112, for example) and the blood port (blood inlet port 105 or blood outlet port 106, for example) which are located at the other end part of the cylindrical housing 109, injecting a potting compound through the blood port (blood outlet port 106, for example) one one end part side and the blood port (blood inlet port 105 or blood outlet port 106, for example) one the other end part side of the cylindrical housing 109 with the end parts of the slender heat-exchanging tubes kept in a closed state, thereby forming partition walls 110a, 110b for fixing the opposite end parts of the slender heat-exchanging tubes 103 to the cylindrical housing 109, removing the first sealing member and the second sealing member, and fitting end plates 108a, 108b one each to the opposite ends of the cylindrical housing 109. To be more specific, after the housing 109 has been formed, one end part of the housing 109 is closed fast by having the sealing member fitted to the end part of the housing 109 at a position (falling between the medium inlet port 113 and the blood outlet port 106, for example) approximating the medium port. The first sealing member is intended to close the end parts of the slender heat-exchanging tubes 103 when the second sealing member is depressed into place as described later on. The sealing members are desired to be formed of an elastic sealing member 158 (a rubber sheet of silicone rubber, polyurethane rubber, or latex rubber, for example) possessing a cross-sectional contour matched to that of the housing 109 as held in the aforementioned place and a retaining device 159 serving to keep the elastic sealing member in place. The elastic sealing member 158 must be incapable of adhering to the potting compound which is poured into the housing 109 later. This requirement may be met by using the potting compound and the elastic sealing member which are made of materials devoid of adhesion quality (silicone rubber sheet for the elastic sealing member and polyurethane as the potting compound or silicone rubbery as the potting compound and polyurethane for the elastic sealing member, for example) or, where the materials therefor both possess adhesive quality, by coating the surface of the elastic sealing member 158 falling on the inner surface side of the housing 109 with resin of the kind capable of cancelling the adhesive quality of the materials (such oil as silicone oil for example). The end parts of the housing 109, as illustrated in Fig. 5 and Fig. 10, are radially diverged in the direction of their extremities from the positions approximating the medium ports 112, 113. The end part of the housing 109 is closed by having the elastic sealing member 158 fitted into the radially diverged portion 160. The possible separation of the elastic sealing member 158 from the housing is precluded by the retaining device 159.

Subsequently, the housing 109 is held upright on the side thereof provided with the aforementioned retaining device 159 and the slender tube-dispersing device 157 is inserted in the housing 109. The slender tube-dispersing device is intended to impart a state pattern to the bundle of slender tubes 103 to be held in a mutually separated state and, therefore, provided with a multiplicity of holes for insertion of the slender tubes 103. In the embodiment illustrated in Fig. 10, the slender tube-dispersing device 157 comprises a plate 161 possessing a multiplicity of holes matched to the slender tubes 103 in a bundled state and a multiplicity of pipes 162 having the end parts thereof inserted in the holes of the plate 161. The pipes 162 are fixed in the plate 161. The pipes 162 have an inside diameter larger than the outside diameter of the slender tubes 103 so that the slender tubes 103 can be inserted into the pipes 162. The largest part of the plate 161 has an outside diameter larger than the inside diameter of the middle part of the housing 109 and smaller than the radially diverged portion 163 so that when this plate is inserted into the housing 109, it will be hung down from the radially diverged portion 163 as illustrated in Fig. 10. The pipes 162 have a length so adjusted that they will not reach the blood outlet port 106 located below when the slender tube-dispersing device 157 is inserted into the housing 109 (namely hung down from the radially diverged portion 163 with the plate 161). This adjustment of length is necessary for the purpose of preventing the pipes 162 of the slender tube-dispersing device 157 from being fixed with the potting compound which will be poured in through the blood outlet port 106 later on. The slender tube-dispersing device using the pipes in the manner described above permits the bundle of slender tubes to be dispersed certainly in a desired pattern. Further, owing to the use of such pipes as mentioned above, the slender tubes 103 and the slender tube-dispersing device 157 contact each other in large portions enough for the slender tubes to be steadily retained in a dispersed state and to be prevented from otherwise possible disruption of the dispersed state under the impact of the potting compound during the injection of the potting compound. After the insertion of the slender tube-dispersing device 157, the slender tubes 103 are inserted into the pipes 162 which have been already inserted through the holes of the plate 161 of the dispersing device 157. The slender tubes 103 thus inserted have the lower end parts thereof thrust out of the leading ends of the pipes 162. The leading ends of the slender tubes 103 come into contact with the elastic sealing member 158. The slender tubes 103 have a length so adjusted that the upper ends thereof fall in the neighborhood of the radially diverged portion of the housing 109. Then, the second sealing member is fitted downwardly into the radially diverged portion 165 of the housing 109 to seal the other end part of the housing 109. The second sealing member, similarly to the first sealing member, is desirably comprising an elastic sealing member 166 possessing a cross-sectional contour matched to that of the housing 109 held in the place mentioned above and a retaining device 166a adapted to keep the elastic sealing member 166 in place. This sealing member is advantageously made of the same material as mentioned above. When the retaining device 166a is depressed into place from above, the end parts of the slender tubes 103 are closed with the elastic sealing members 158, 166. The heat exchanger has been so far described as being provided with the elastic sealing member 166. Since it is only required to close the end parts of the slender heat-exchanging tubes, the installation thereof is not necessarily indispensable up to this point in the whole course of the production. Then, the potting compound is poured in through the blood outlet port 106 and allowed to set. Subsequently, the upper retaining device 166a, the elastic sealing member 166, and the slender tube-dispersing device 157 are removed. Further, the elastic sealing member 166 is again fitted in the radially diverged portion 165 of the housing 109 to close the end of the housing 109 and set in place with the retaining device 166a. Now, the housing 109 is turned upside down and the retaining device 159 is depressed into place from above to close the end parts of the slender tubes 109. In the same manner as described above, the potting compound is poured in through the blood outlet port 106 (or the blood inlet port 105) and allowed to set. After the potting compound has been solidified, the retaining devices 159, 166a and the elastic sealing members 158, 166 attached to the opposite end parts of the housing 109 are removed. Consequently, the partition walls 110a, 110b are formed.

Since the method described above requires the removal of the slender tube-dispersing device as one of the essential steps thereof, the injection of the potting compound can be attained infallibly and easily even on the side used for the insertion of the slender tube-dispersing device 157. Since the opposite ends of the slender tubes 103 are kept in place with the elastic sealing members 158, 166, the elastic sealing members absorb any possible minor variation of length of the slender tubes 103 and are capable of preventing the potting compound from flowing into the slender tubes 103. Further, the end plates 108a, 108b possessing a cross-sectional contour matched to that of the opposite ends of the housing 109 are fixed watertightly to the opposite end parts of the housing 109. The fixation of the end plates is attained by adhesion with adhesive agent or by fusion with high-frequency waves, ultrasonic waves, or induction heating.

The heat exchanger has been so far described as being provided with sealing members comprising elastic sealing members 158, 166 and retaining devices 159, 166a. Optionally, the sealing members used for the heat exchanger may be of the type produced by integrally forming the elastic sealing members and the retaining devices.

The insertion into the cylindrical housing 109 through the other end part of the cylindrical housing 109 of the slender tube-dispersing device possessing multiplicity of holes of insertion of slender heat-exchanging tubes and the multiplicity of slender heat-exchanging tubes may be carried out with the housing kept in a horizontal state. The insertion carried out with the housing kept in an upright state proves to be more desirable because the slender tubes can be inserted with greater ease. The insertion into the cylindrical housing 109 through the other end part of the cylindrical housing 109 of the slender tube-dispersing device possessing multiplicity of holes for insertion of slender heat-exchanging tubes and the multiplicity of slender heat-exchanging tubes may be effected, for example, by first inserting the slender tubes 103 through the holes of the slender tube-dispersing device 157 and then causing the slender tube-dispersing device 157 now holding the slender tubes 103 inserted therethrough to be inserted in the housing 109. The formation of the partition walls may be carried out, for example, by rotating the housing 109 about the axis thereof while keeping the end parts of the slender heat-exchanging tubes 103 fast in place, then centrifugally pouring the potting compound through the blood port on one end part side of the cylindrical housing 109, allowing the introduced potting compound to set, and repeating the same procedure on the other end side. Otherwise, it may be effected by rotating the housing about the central part of the housing and, at the same time, centrifugally pouring the potting compound simultaneously through the blood ports on the opposite end parts and allowing the introduced potting compound to set.

The step of fitting the end plates 108a, 108b, to the opposite ends of the housing 109 may comprise first removing either the first sealing member or the second sealing member, fitting one of the end plates to the end part on the side on which the removal just mentioned has been made, removing the remaining sealing member, and fitting the other end plate to the end part on the side on which the removal has been made.

The method for production has been so far described as requiring the removal of the slender tube-dispersing devices as one of the component steps. When the slender tube-dispersing device 157 is such that the continued presence thereof within the housing offers no hindrance whatever to the use of the heat exchanger, it need not be removed by all means. As a concrete example of the slender tube-dispersing device 157 which answers the description, a plate formed of a metallic substance such as stainless steel or a synthetic resin as illustrated in Fig. 11 may be cited. This slender tube-dispersing device 157 is provided with a plate part 161 possessing a multiplicity of holes permitting insertion thereof of the slender tubes 103 and a plurality (at least three) of leg parts 161a extended downwardly from the plate part 161. The plate part 161 has a lateral part thereof cut away to permit downward flow of the potting compound (fig 11) When the slender tube-dispersing device of this construction is used in pouring the potting compound through the blood port, it is eventually buried in the partition wall to be formed of the potting compound as illustrated on fig. 11 Thus, this slender tube-dispersing device places no hindrance whatever in the way of actual use of the heat exchanger.

The method for the production of the heat exchanger of this invention comprises the steps of forming a cylindrical housing possessing heat-exchanging medium ports disposed one each at the opposite end parts thereof and a blood inlet port and a blood outlet port disposed at positions between the medium ports, attaching a first sealing member at a position between those of the medium ports and the blood ports which are located at one end part of the cylindrical housing, inserting thourgh the other end part of the cylindrical housing into the cylindrical housing a slender tube distributing device possessing a multiplicity of holes for insertion of slender heat-exchanging tubes and a multiplicity of slender heat-exchanging tubes, attaching a second sealing member at a position between those of the medium ports and the blood ports which are located at the other end part of the cylindrical housing, injecting a potting compound through the blood port on one end part side and the blood port on the other end part side of the cylindrical housing with the end parts of the slender heat-exchanging tubes kept in a closed state thereby forming partition walls for fixing the opposite end parts of the slender heat-exchanging tubes to the cylindrical housing, removing the first sealing member and the second sealing member, and fitting end plates one each to the opposite ends of the cylindrical housing. By this method, the heat exchanger of this invention described above can be easily produced.

## Claims

1. A method for manufacturing a medical heat exchanger, which comprises the steps of introducing a multiplicity of slender heat-exchanging tubes (103) into a cylindrical housing (109) possessing a heat-exchanging medium inlet port (113), a heat-exchanging medium outlet port (112), a blood inlet port (105) and a blood outlet port (106); fitting first and second sealing members to the end parts of the housing (109); injecting a potting compound into the cylindrical housing (109) with the end parts of the slender heat-exchanging tubes (103) kept in closed state thereby forming partition walls (110a, 110b) for fixing each end part of the slender heat-exchanging tubes (103) to the cylindrical housing (109); and removing the first and second sealing members (158, 166), characterized in that the potting compound is injected through the blood inlet port (105) and/or the blood outlet port (106) which are disposed at positions between said medium ports (112, 113); the sealing members comprise elastic sealing members (158, 166) capable of closing the end parts of the slender heat-exchanging tubes (103) and are attached at respective positions between said medium port and blood port to both end parts of the cylindrical housing; the slender heat-exchanging tubes (103) are introduced into the housing (109) aided with a slender tube-distributing device (157) possessing a multiplicity of holes for insertion of the slender heat-exchanging tubes (103); end plates (108a, 108b) are finally fitted on the opposite ends of the cylindrical housing.

2. A method according to claim 1, wherein said slender tube-distributing device (157) and said slender heat-exchanging tubes (103) are introduced into said cylindrical housing (109) through one end part of said cylindrical housing with said cylindrical housing (109) kept standing upright on the other end part thereof provided with said first sealing member (158).

3. A method according to claim 1, wherein said slender tube-distributing device (157) and said slender heat-exchanging tubes (103) are introduced into said cylindrical housing (109) through one end part of said cylindrical housing by first inserting said slender tube-distributing device (157) and subsequently inserting said multiplicity of slender heat-exchanging tubes (103).

4. A method according to claim 1 wherein the step of injecting said potting compound is effected by first pouring said potting compound through said blood outlet port (106) on one end part side of said cylindrical housing (109), allowing the poured potting compound to set, turning said cylindrical housing (109) upside down, pouring said potting compound through said blood inlet port (105) on the other end part side of said cylindrical housing (109), and allowing the poured potting compound to set.

5. A method according to claim 1 wherein the step of injecting said potting compound is effected by pouring said potting compound through said blood outlet port (106) on one end part side of said cylindrical housing, allowing the poured potting compound to set, removing the sealing member (166) fitted to said end part side and said slender tube-distributing device (157), fitting again said sealing member (166) in place, turning the cylindrical housing (109) upside down and pouring said potting agent through said blood outlet port (106) or the blood inlet port (105) while keeping the end parts of said slender heat exchanging tubes in a sealed state and allowing the poured potting compound to set.

6. A method according to claim 1, wherein the step of removing said first and second sealing members (158, 166) and the step of fitting end plates (108a, 108b) on opposite ends of said cylindrical housing comprises first removing said sealing members (158, 166) and subsequently attaching said end plates (108a, 108b) on the opposite end parts of said cylindrical housing.

7. A method according to claim 1 wherein the step of removing said first and second sealing members (158, 166) and the step of fitting end plates (108a, 108b) on each opposite ends of said cylindrical housing comprises first removing either the first sealing member (158) or the second sealing member (166), attaching an end plate (108a) to the end part of the side on which said first removal is effected, removing the remaining sealing member (166) and attaching another end plate (108b) to the end part of the side on which said second removal is effected.

8. A method according to claim 1, further comprising the step of keeping in place the first sealing member (158) by a retaining device (159) whereby the end parts of the slender heat-exchanging tubes (103) are closed by said first sealing member (158).

9. A method according to claim 8, further comprising the step of keeping in place the second sealing member (166) by a retaining device (166a).

10. A method according to claim 1, wherein said slender tube-distributing device (157) comprises a plate (161) having a multiplicity of holes and a multiplicity of pipes (162) having an inside diameter larger than the inside diameter of the slender heat-exchanging tubes (103), said pipes (162) having a length so adjusted that the pipes are prevented from being fixed with the potting compound upon injection thereof.

11. A method according to claim 1 or 10 wherein the slender tube-distributing device (157) is removed after injection of the potting compound.

12. A method according to claim 1 wherein the slender tube-distributing device (157) is not removed from the housing, and comprises a plate (161) having a multiplicity of holes, the plate (161) having a lateral part thereof cut away to permit flow of the potting compound.

13. A method according to claim 12 wherein means (161a, 556) are provided for keeping the plate (161, 555) apart from said sealing member (166).

## Patentansprüche

1. Verfahren zur Herstellung eines medizinischen Wärmetauschers, umfassend die Schritte des Einführens einer Vielzahl von schlanken Wärmeaustauschrohren (103) in ein zylindrisches Gehäuse (109), das eine Einlaßöffnung (113) für ein Wärmeaustauschmedium, eine Auslaßöffnung (112) für ein Wärmeaustauschmedium, eine Bluteinlaßöffnung (105) und eine Blutauslaßöffnung (106) besitzt; des Einsetzens eines ersten und eines zweiten Dichtelements an den Endteilen des Gehäuses (109); des Einspritzens einer Vergießverbindung in das zylindrische Gehäuse (109), wobei die Endteile der schlanken Wärmeaustauschrohre (103) in geschlossenem Zustand gehalten werden, so daß Trennwände (110a, 110b) zum Befestigen jedes Endteils der schlanken Wärmeaustauschrohre (103) an dem zylindrischen Gehäuse (109) gebildet werden; und des Entfernens des ersten und des zweiten Dichtelements (158, 166), dadurch gekennzeichnet, daß die Vergießverbindung durch die Bluteinlaßöffnung (105) und/oder die Blutauslaßöffnung (106) eingespritzt wird, die an Positionen zwischen den Mediumöffnungen (112, 113) angeordnet sind; die Dichtelemente elastische Dichtelemente (158, 166) umfassen, die in der Lage sind, die Endteile der schlanken Wärmeaustauschrohre (103) zu verschließen und an jeweiligen Positionen zwischen der Mediumöffnung und der Blutöffnung an beiden Endteilen des zylindrischen Gehäuses angebracht werden; die schlanken Wärmeaustauschrohre (103) in das Gehäuse (109) mit Hilfe einer Verteileinrichtung (157) für schlanke Rohre eingeführt werden, die eine Vielzahl von Löchern zum Einführen der schlanken Wärmeaustauschrohre (103) besitzt; Endplatten (108a, 108b) schließlich an den entgegengesetzten Enden des zylindrischen Gehäuses angesetzt werden.

2. Verfahren nach Anspruch 1, bei welchem die Verteileinrichtung (157) für schlanke Rohre und die schlanken Wärmeaustauschrohre (103) in das zylindrische Gehäuse (109) durch einen Endteil des zylindrischen Gehäuses eingeführt werden, wobei das zylindrische Gehäuse (109) aufrechtstehend auf seinem anderen Endteil gehalten wird, der mit dem ersten Dichtelement (158) versehen ist.

3. Verfahren nach Anspruch 1, bei welchem die Verteileinrichtung (157) für schlanke Rohre und die schlanken Wärmeaustauschrohre (103) in das zylindrische Gehäuse (109) durch einen Endteil des zylindrischen Gehäuses eingeführt werden, indem zuerst die Verteileinrichtung (157) für schlanke Rohre eingeführt wird und anschließend die Vielzahl von schlanken Wärmeaustauschrohren (103) eingeführt werden.

4. Verfahren nach Anspruch 1, bei welchem der Schritt des Einspritzens der Vergießverbindung ausgeführt wird, indem zunächst die Vergießverbindung durch die Blutauslaßöffnung (106) auf der Seite eines Endteils des zylindrischen Gehäuses (109) eingegossen wird, das Aushärten der eingegossenen Vergießverbindung zugelassen wird, das zylindrische Gehäuse (109) umgedreht wird, die Vergießverbindung durch die Bluteinlaßöffnung (105) auf der Seite des anderen Endteils des zylindrischen Gehäuses (109) eingegossen wird und das Aushärten der eingegossenen Vergießverbindung zugelassen wird.

5. Verfahren nach Anspruch 1, bei welchem der Schritt des Einspritzens der Vergießverbindung ausgeführt wird, indem die Vergießverbindung durch die Blutauslaßöffnung (106) auf der Seite eines Endteils des zylindrischen Gehäuses eingegossen wird, das Aushärten der eingegossenen Vergießverbindung zugelassen wird, das Dichtelement (166), das an der Seite des Endteils angebracht ist, und die Verteileinrichtung (157) für schlanke Rohre entfernt werden, erneut das Dichtelement (166) an seine Position gesetzt wird, das zylindrische Gehäuse (109) umgedreht wird und die Vergießverbindung durch die Blutauslaßöffnung (106) oder die Bluteinlaßöffnung (105) gegossen wird, während die Endteile der schlanken Wärmeaustauschrohre in einem abgedichteten Zustand gehalten werden und das Aushärten der eingegossenen Vergießverbindung ermöglicht wird.

6. Verfahren nach Anspruch 1, bei welchem der Schritt des Entfernens des ersten und des zweiten Dichtelements (158, 166) und der Schritt des Ansetzens der Endplatten (108a, 108b) an entgegengesetzten Enden des zylindrischen Gehäuses zunächst das Entfernen der Dichtelemente (158, 166) und anschließend das Anbringen der Endplatten (108a, 108b) an den entgegengesetzten Endteilen des zylindrischen Gehäuses umfaßt.

7. Verfahren nach Anspruch 1, bei welchem der Schritt des Entfernens des ersten und des zweiten Dichtelements (158, 166) und der Schritt des Ansetzens der Endplatten (108a, 108b) an jedem der entgegengesetzten Enden des zylindrischen Gehäuses zunächst das Entfernen entweder des ersten Dichtelements (158) oder des zweiten Dichtelements (166), das Anbringen einer Endplatte (108a) an dem Endteil der Seite, an welcher das erste Entfernen ausgeführt wird, das Entfernen des verbleibenden Dichtelements (166) und das Anbringen einer weiteren Endplatte (108b) an dem Endteil der Seite, an welchem das zweite Entfernen ausgeführt wird, umfaßt.

8. Verfahren nach Anspruch 1, ferner umfassend den Schritt des Festhaltens des ersten Dichtelements (158) an seiner Position durch eine Rückhalteeinrichtung (159), wodurch die Endteile der schlanken Wärmeaustauschrohre (103) durch das erste Dichtelement (158) geschlossen werden.

9. Verfahren nach Anspruch 8, ferner umfassend den Schritt des Haltens des zweiten Dichtelements (166) an seiner Position durch eine Rückhalteeinrichtung (166a).

10. Verfahren nach Anspruch 1, bei welchem die Verteileinrichtung (157) für schlanke Rohre eine Platte (161) umfaßt, die eine Vielzahl von Löchern und eine Vielzahl von Rohren (162) hat, die einen Innendurchmesser haben, der größer ist als der Innendurchmesser der schlanken Wärmeaustauschrohre (103), welche Rohre (162) eine Länge haben, die so eingestellt ist, daß verhindert wird, daß die Rohre mit der Vergießverbindung bei deren Einspritzen fixiert werden.

11. Verfahren nach Anspruch 1 oder 10, bei welchem die Verteileinrichtung (157) für schlanke Rohre nach dem Einspritzen der Vergießverbindung entfernt wird.

12. Verfahren nach Anspruch 1, bei welchem die Verteileinrichtung (157) für schlanke Rohre nicht aus dem Gehäuse entfernt wird und eine Platte (161) umfaßt, die eine Vielzahl von Löchern hat, welche Platte (161) einen ausgeschnittenen seitlichen Teil hat, um das Fließen der Vergießverbindung zu erlauben.

13. Verfahren nach Anspruch 12, bei welchem eine Einrichtung (161a, 556) vorgesehen ist, um die Platte (161, 555) von dem Dichtelement (166) getrennt zu halten.

## Revendications

1. Procédé de fabrication d'un échangeur de chaleur médical, comprenant les étapes consistant: à introduire une multiplicité de tubes fins (103) d'échange de chaleur dans une enveloppe cylindrique (109) comportant un orifice (113) d'entrée de fluide d'échange de chaleur, un orifice (105) d'entrée de sang, un orifice (112) de sortie de fluide d'échange de chaleur, un orifice (106) de sortie de sang; à monter des premier et deuxième éléments d'étanchéité sur les parties d'extrémité de l'enveloppe (109); à injecter une composition d'enrobage dans l'enveloppe cylindrique (109), les parties d'extrémité des tubes fins (103) d'échange de chaleur étant maintenues dans un état fermé, en formant ainsi des parois de séparation (110a, 110b) destinées à fixer chaque partie d'extrémité des tubes fins (103) d'échange de chaleur à l'enveloppe cylindrique (109); et à enlever les premier et deuxième éléments d'étanchéité (158, 166), caractérisé en ce que on injecte la composition d'enrobage à travers l'orifice (105) d'entrée de sang et/ou à travers l'orifice (106) de sortie de sang qui se trouvent à des endroits situés entre lesdits orifices (112, 113) de passage de fluide d'échange de chaleur; les éléments d'étanchéité comprennent des éléments d'étanchéité élastiques (158, 166) capables de fermer les parties d'extrémité des tubes fins (103) d'échange de chaleur et on les fixe à des endroits respectifs situés entre ledit orifice de passage de fluide d'échange de chaleur et l'orifice de passage de sang aux deux parties d'extrémité de l'enveloppe cylindrique; on introduit les tubes fins (103) d'échange de chaleur dans l'enveloppe (109) en étant assisté par un dispositif de répartition de tubes fins (157) comportant une multiplicité de trous destinés à l'insertion des tubes fins (103) d'échange de chaleur; on monte finalement des plaques d'extrémité (108a, 108b) sur les extrémités opposées de l'enveloppe cylindrique.

2. Procédé selon la revendication 1, dans lequel on introduit ledit dispositif (157) de répartition de tubes fins et lesdits tubes fins (103) d'échange de chaleur dans ladite enveloppe cylindrique (109) à travers une des parties d'extrémité de ladite enveloppe cylindrique , ladite enveloppe cylindrique (109) étant maintenue verticale sur son autre partie d'extrémité munie dudit premier élément d'étanchéité (158).

3. Procédé selon la revendication 1, dans lequel on introduit ledit dispositif (157) de répartition de tubes fins et lesdits tubes fins (103) d'échange de chaleur dans ladite enveloppe cylindrique (109) à travers une des parties d'extrémité de ladite enveloppe cylindrique en insérant tout d'abord ledit dispositif (157) de répartition de tubes fins et en insérant ensuite ladite multiplicité de tubes fins (103) d'échange de chaleur.

4. Procédé selon la revendication 1, dans lequel on effectue l'étape d'injection de ladite composition d'enrobage en versant tout d'abord ladite composition d'enrobage à travers ledit orifice (106) de sortie de sang se trouvant sur un des côtés de partie d'extrémité de ladite enveloppe cylindrique (109), en laissant durcir la composition d'enrobage, en inversant la position verticale de ladite enveloppe cylindrique (109), en versant ladite composition d'enrobage à travers ledit orifice (105) d'entrée de sang se trouvant sur l'autre côté de partie d'extrémité de ladite enveloppe cylindrique (109), et en laissant durcir la composition d'enrobage versée.

5. Procédé selon la revendication 1, dans lequel on effectue l'étape d'injection de ladite composition d'enrobage en versant ladite composition d'enrobage à travers ledit orifice (106) de sortie de sang se trouvant sur un des côtés de partie d'extrémité de ladite enveloppe cylindrique, en laissant durcir la composition d'enrobage versée, en enlevant l'élément d'étanchéité (166) monté sur ledit côté partie d'extrémité et ledit dispositif (157) de répartition de tubes fins, en montant de nouveau ledit élément d'étanchéité (166) en inversant la position verticale de l'enveloppe cylindrique (109) et en versant ledit agent enrobage à travers ledit orifice (106) de sortie de sang ou l'orifice (105) d'entrée de sang tout en maintenant les parties d'extrémité desdits tubes fins d'échange de chaleur dans un état fermé de façon étanche et en laissant durcir la composition d'enrobage versée.

6. Procédé selon la revendication 1, dans lequel l'étape d'enlèvement desdits premier et deuxième éléments d'étanchéité (158, 166) et l'étape de montage des plaques d'extrémité (108a, 108b) sur les extrémités opposées de ladite enveloppe cylindrique comprennent tout d'abord l'enlèvement desdits éléments d'étanchéité (158, 166) puis la fixation desdites plaques d'extrémité (108a, 108b) sur les parties d'extrémité opposées de ladite enveloppe cylindrique.

7. Procédé selon la revendication 1, dans lequel l'étape d'enlèvement desdits premier et deuxième éléments d'étanchéité (158, 166) et l'étape de montage des plaques d'extrémité (108a, 108b) sur chacune des extrémités opposées de ladite enveloppe cylindrique comprend tout d'abord l'enlèvement soit du premier élément d'étanchéité (158) soit du deuxième élément d'étanchéité (166), la fixation d'une plaque d'extrémité (108a) à la partie d'extrémité du côté sur lequel a été effectué ledit premier enlèvement, l'enlèvement de l'élément d'étanchéité restant (166) et la fixation d'une autre plaque d'extrémité (108b) à la partie d'extrémité du côté sur lequel a été effectué ledit deuxième enlèvement.

8. Procédé selon la revendication 1, comprenant, en outre l'étape de maintien en place du premier élément d'étanchéité (158) par un dispositif de retenue (159), les parties d'extrémité des tubes fins 103) d'échange de chaleur étant ainsi fermées par ledit premier élément d'étanchéité (158).

9. Procédé selon la revendication 8, comprenant, en outre, l'étape de maintien en place du deuxième élément d'étanchéité (166) par un dispositif de retenue (166a).

10. Procédé selon la revendication 1, dans lequel ledit dispositif (157) de répartition de tubes comprend une plaque (161) comportant une multiplicité de trous et une multiplicité de tubulures (162) ayant un diamètre intérieur plus grand que le diamètre intérieur des tubes fins (103) d'échange de chaleur, lesdites tubulures (162) ayant une longueur qui est ajustée de telle sorte que les tubulures ne peuvent pas être fixées par la composition d'enrobage lors de l'injection de cette dernière.

11. Procédé selon la revendication 1 ou 10, dans lequel on enlève le dispositif (157) de répartition de tubes fins après l'injection de la composition d'enrobage.

12. Procédé selon la revendication 1, dans lequel le dispositif (157) de répartition de tubes fins n'est pas enlevé de l'enveloppe et comprend une plaque (161) comportant une multiplicité de trous, la plaque (161) comportant une partie latérale échancrée pour permettre l'écoulement de la composition d'enrobage.

13. Procédé selon la revendication 12, dans lequel des moyens (161a, 556) sont prévus pour maintenir la plaque (161, 555) écartée dudit élément d'étanchéité (166).
